# EUROPEAN PATENT SPECIFICATION

(11) **EP 3 346 907 B1**
(45) Date of publication and mention of the grant of the patent: **29.01.2020**
(21) Application number: 16781583.6
(22) Date of filing: 29.08.2016
(51) Int. Cl.: A61B 5/00, A61B 5/145

(54) **A UREA, PHOSPHATE AND PH MEASURING DEVICE**
MESSVORRICHTUNG FÜR HARNSTOFF, PHOSPHAT UND PH-WERT
DISPOSITIF DE MESURE D'URÉE, DE PHOSPHATE ET DE PH

(30) Priority: 07.09.2015 TR 201511075
(43) Date of publication of application: 18.07.2018
(73) Proprietor: Isildak, Ibrahim, Esenler/Istanbul (TR); Tavukcuoglu, Ozlem, Kadikoy/Istanbul (TR); Nigde, Mustafa, Pendik/Istanbul (TR); Yildirim, Ridvan, Pendik/Istanbul (TR); Agir, Ismail, Bahcelievler/Istanbul (TR)
(72) Inventor: Isildak, Ibrahim, Esenler/Istanbul (TR); Tavukcuoglu, Ozlem, Kadikoy/Istanbul (TR); Nigde, Mustafa, Pendik/Istanbul (TR); Yildirim, Ridvan, Pendik/Istanbul (TR); Agir, Ismail, Bahcelievler/Istanbul (TR)
(74) Representative: Cayli, Hülya
(86) International application number: PCT/TR2016/050313
(87) International publication number: WO 2017/044063

(56) References cited:
- WO-A1-98/28613
- US-A- 4 713 165
- US-A1- 2005 214 161
- US-A1- 2011 201 383

## Description

### Related Technical Field of the Invention:

This invention is related with a device which measures urea, phosphate and pH values in a little amount of blood at the same time.

### Prior Art:

The chronic renal failure is a clinical disease which appears as a result of loss of nephrons progressively and irremediably due to a reason related or not related with kidneys. Liquid, acid-base and electrolyte balance disorders are frequent problems in the renal failure. In case of high level loss of kidney functions; to remove phosphate and urea from the body fluid cannot be provided and dialysis is required. Therefore; frequent monitoring of urea, phosphate (PO₄³⁻) and pH parameters in blood through many years has a significant importance in order to determine the levels of uremia, hyperphosphatemia and acid-base balance disorder in the renal failure patients. Providing diet according to urea, phosphate and pH values via monitoring these values in the blood of patient people who are in the dialysis process or who does not need dialysis yet, but has reduced kidney functions can minimize long term negative effects which occur because of the disease in these patients.

Nowadays, the dialysis patients need to go to hospitals or dialysis centers in order to learn the urea, phosphate and pH values in their blood.

Determination of urea, phosphate and pH is performed generally via desktop systems in hospitals and dialysis centers. This situation requires that patients go to the hospital or dialysis center periodically and affects the life quality of the patient negatively. As a result of these measurements which are performed frequently, dialysis patients can live more comfortably by controlling their diet.

In addition, blood samples in high volumes are generally taken from patients via invasive application from vascular access for urea, phosphate and pH measurents in said methods used in the state of the art.

Nowadays, there is not any system or device from which patients can learn their said values without depending on a hospital, laboratory or dialysis center. Blood urea value is measured via spectrophotometric, enzymatic and kinetic methods; phosphate value is measured via the spectrophotometric method; pH value is measured via desktop systems with special electrodes in blood gas analyzers.

At the present time, another method which can be used for determination of different analytes is the potentiometric method. Potentiometry is a determination method in which the changing potential difference between the ion selective sensor and reference electrode is measured depending on the activities of the ions in the medium in case that there is no current or very little current passing through the solution in a electrochemical cell.

The ion concentration is measured directly by considering the changing potential of the ion selective sensor in the potentiometry. The potentiometric system consists of a test cell (electrolytic solution), ion selective sensor or molecule selective biosensor (variable potential) which is connected to it, reference electrode (constant potential) and a stable potentiometer.

An ion selective sensor works together with at least one reference electrode in the potentiometric measurements.

In the patent document with the number of US8273228B2 at the state of the art, a portable device which provides measurement and control by using biosensors in the determination of glucose and other analytes in the biological fluids is described.

In the patent document with the number of US5711862 at the state of the art, a biochemical enzyme sensor which measures the glucose value voltametrically is described.

In the patent document with the number of US2006040251 at the state of the art, a device which can measure one single agent like glucose via the voltametric method is described.

Patent document US4713165A discloses a sensor for the potentiometric determination of the ion content or activity of a sample. Said sensor comprises ion-selective electrodes which are held in a frame and have porous material between them. Each of said ion-selective electrodes comprises a membrane which is selectively permeable to the ion or other substance whose activity or concentration is being determined; a reference electrode; and an internal reference material. In this document, each of the ion-selective electrodes contains a chamber for storing the sample to be analysed. According to the this document, by analysing the sample, amounts of hydrogen, ion glucose, urea, triglycerides, uric acid enzymes such as aspartate aminotransferase (AST), alanine aminotransferase (ALT), amylase, creatinine kinase (CK), alkaline phosphatase and drugs can be detected.

### Brief Description of the Invention:

The aim of this invention is a urea, phosphate and pH measuring device which can perform the measurement of urea, phosphate and pH values in blood at the same time with a little amount of blood sample.

The disclosed measurement device of the invention can be carried easily by the patient or user and can measure urea, phosphate and pH values in a little amount of blood taken from finger in every environment.

The disclosed measurement device of the invention is defined by claim 1 and comprises at least one control module, at least one measurement division and at least one communication module.

The control module is a unit which determines the urea, phosphate and pH value in blood simultaneously and correctly and provides its presentation to the user by comparing the values determined and the reference values.

The control module is a module which presents to the user if the measurement values which it takes from the urea sensing unit, phosphate sensing unit and pH sensing unit are in the interval that healthy humans need to have by comparing said data with the reference values and how much they are lower or higher if they are not in the value interval that healthy humans need to have by controlling them.

The control module provides that emergency information is given to the user in case that the values measured are lower or higher than the value interval of healthy humans.

The measurement module comprises at least one urea sensing unit for determining urea amount in blood, at least one phosphate sensing unit for determining phosphate amount in blood, at least one pH sensing unit for determining hydronium ion level in blood and a reference electrode unit.

The measurement module is in the "strip" form comprising at least one urea sensing unit, at least one phosphate sensing unit and at least one pH sensing unit.

The strip comprises a sample division in which the blood sample is positioned.

The sample division comprises wettable polymer, a hydrogel or cellulosic filtration layer which provide filtration of the blood sample positioned.

The filtration layer comprises a solution which performs calibration for measurements of urea, phosphate and pH.

The solution consists of the components whose urea, phosphate and pH levels are known.

The urea sensing unit comprises at least one urea biosensor which has the urease enzyme for determination of urea amount in blood.

The urea biosensor detects ammonium ion changes which occur as a result of hydrolysis reaction catalyzed by urease.

The phosphate sensing unit comprises at least one phosphate sensor which is in composite structure comprising an ionophore which is sensitive for HPO₄²⁻ (hydrogen phosphate) ion, epoxy, hardener and modified graphite or graphene for determining phosphate amount in blood.

pH sensing unit comprises at least one composite structured pH sensor which can measure pH value for determination acidity or alkalinity of the patient blood, which is necessary for correction of urea and phosphate amounts at the same time.

The reference unit comprises at least one solid-state reference electrode which shows constant potential in the sample solution for determination of urea, phosphate concentrations and pH in the blood of patient.

The communication module is the module which can provide remote access via a smart device, can also conduct the transmission of data obtained in consequence of the measurements to the patient remotely via wireless connections.

The communication module comprises the unit that provides transmission of data which is received from the measurement module and whose comparison and calculation is performed by the control unit to the patient and/or doctor.

The communication module comprises a sim card which provides transmission of data obtained in consequence of the measurement to the patient and/or doctor.

The disclosed measurement device of the invention comprises a display which shows the values obtained in consequence of the measurement to the patient.

Due to the disclosed measurement device of the invention; the patients who receives dialysis treatment can monitor the urea, phosphate and pH values with a little volume of blood sample which they take from finger on their own without going to hospitals or dialysis centers.

In addition, the disclosed measurement device of the invention can be applied to the dialysis patients also by health officers in hospitals easily. It provides to get result faster and more practical than the desktop measurement systems used in the processes before the disclosed device of the invention.

Additionally, it is not necessary to enter to the vascular access via needle for determination of the levels of urea, phosphate and pH agents in the patient blood due to the disclosed device of the invention. The life quality of patients is increased by providing them not to suffer due to a little amount of blood sample taken from finger.

In addition to patients' own monitoring of said agents in their blood via the disclosed urea, phosphate and pH measuring device of the invention; the results obtained can be sent to the related doctor and that's why, time and duration of dialysis can be decided earlier effectively.

Hyperphosphatemia and uremic syndromes which occur in the chronic renal failure disease may cause life threat for these patients quickly. Slowing down of threat can be provided via dialysis and diet applications. Therefore, to follow the patients who have chronic renal failure closely has importance. This situation requires that urea and phosphate blood values which cause uremia and phosphatemia are measured frequently. That's why, the disclosed measurement device of the invention provides that blood urea, phosphate levels of the patients who have a chronic renal failure threat and in addition, blood pH level which is closely related to these parameters are measured effectively.

The disclosed measurement device of the invention can be applied easily and comfortably by the patient at home with a high originality, sensitivity and accuracy percentage for monitoring uremic syndrome and hyperphosphatemia in renal failure patients effectively.

Due to the disclosed urea, phosphate and pH measurement device of the invention; the patients can determine their diet and treatment regimen on their own or with the doctor deliberatively, not randomly, by considering levels of these agents which they monitor.

### Detailed Description of the Invention Description of the Figures

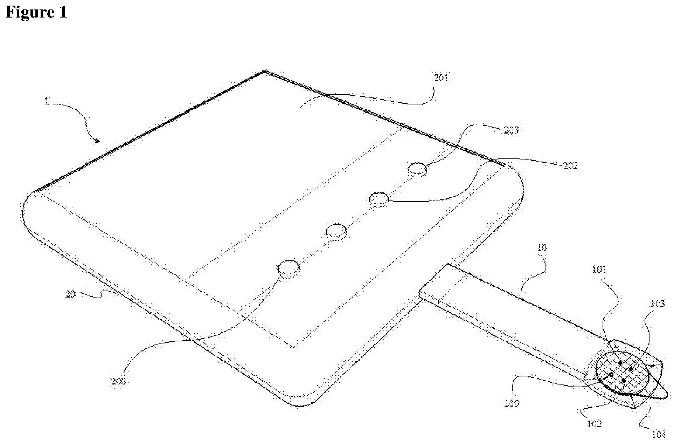

**Figure 1****-** It is the view of the disclosed measurement device of the invention.

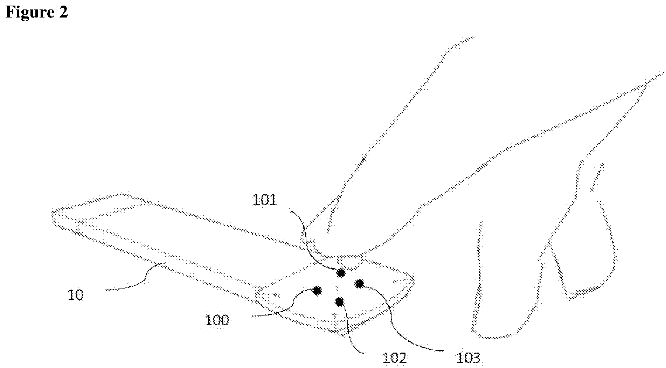

**Figure 2****-** It is the view of the disclosed measurement device of the invention during its application.

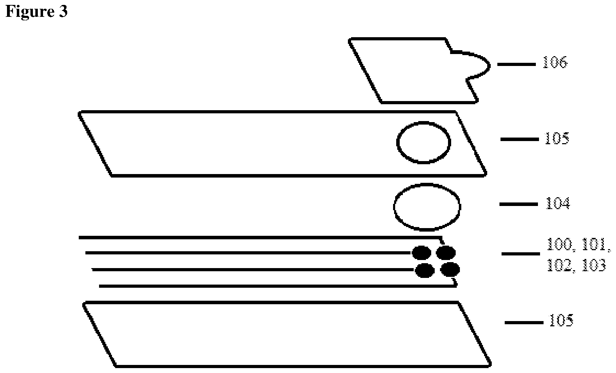

**Figure 3****-** It is the view of the strip type measurement module of the disclosed measurement device of the invention.

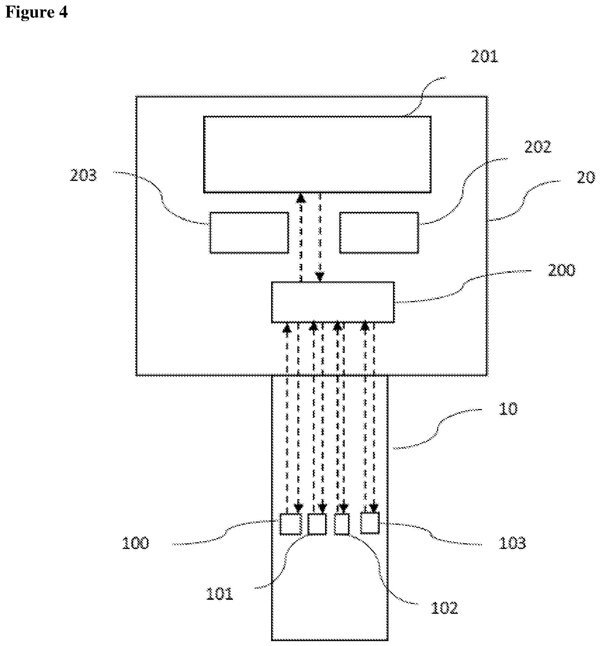

**Figure 4****-** It is the block diagram of the disclosed device of the invention.

### Description of the references in the Figures:

The parts in the figures are numbered respectively and the equivalents of these numbers are given below.
1. Urea, phosphate and pH measuring device
10. Measurement module
   100. Urea sensing unit
   101. Phosphate sensing unit
   102. pH sensing unit
   103. Reference unit
   104. Sampling and filtration unit
   105. Plastic layer
   106. Removable plastic cover
20. Control module
   200. Control unit
   201. Display
   202. Power unit
30. Communication module

The disclosed measurement device (1) of the invention comprises a measurement module (10) in which urea, phosphate and pH measurements are performed in the blood taken from the patient, a control module (20) in which said results are calculated and shown to the patient and a communication module (30) which can provide remote access.

The disclosed measurement device (1) of the invention works potentiometrically.

The measurement module (10) is in the strip form.

The strip shaped measurement module (10) comprises at least one urea sensing unit (100) which performs urea measurement, at least one phosphate sensing unit (101) which performs phosphate measurement and at least one pH sensing unit (102) which performs pH measurement.

The urea sensing unit (100) comprises an entire solid-state contact ammonium selective sensor. There is the urease enzyme as a biolayer on the sensor surface. Urease is an enzyme which is special for urea. The urease enzyme releases ammonium ion to the environment by giving reaction with urea. The urea sensing unit (100) provides determination of the urea concentration in blood by finding the concentration of ammonium ion released. In another embodiment of the invention, the urea sensing unit (100) can perform the ammonium measurement process also via another enzyme.

In the embodiment of the invention, the urea sensing unit (100) comprises at least one urea biosensor which provides determination of urea for finding the urea amount in blood.

The phosphate sensing unit (101) comprises a special composite material comprising a phosphate complex which is sensitive to HPO₄²⁻ ion. Determination of the phosphate concentration in blood is provided via determination of the concentration of this ion.

In the embodiment of the invention, the phosphate sensing unit (101) comprises at least one phosphate sensor for determining the amount of phosphate ion in blood.

pH sensing unit (102) comprises a special composite material comprising quinhydron which is sensitive to H₃O⁺ ion. pH sensing unit (102) comprises at least one pH sensor for determination of the pH of blood in the embodiment of the invention.

The strip shaped measurement module (10) also comprises a reference unit (103). The reference unit (103) comprises at least one Ag/AgCl based composite structured reference electrode used in providing urea, phosphate and pH measurements in blood.

In an embodiment of the invention, reference electrode does not participate in the measurements. It acts as reference by producing a constant potential in the blood sample for performing urea, phosphate and pH measurements correctly.

In the disclosed device (1) of the invention performing urea, phosphate and pH measurements, only one reference electrode which is connected in series with the urea, phosphate and pH sensors works.

The measurement module (10) comprises a sampling and filtration unit (104) which the blood sample whose urea, phosphate and pH values are to be measured is dropped into. It is provided that the filtration unit absorbs a calibration solution which provides sensors to measure reliably.

The measurement module (10) comprises two plastic layers (105).

The measurement module (10) comprises a plastic cover (106) which can be removed and thrown away. The removable plastic cover (106) is to provide conservation of the calibration solution absorbed into the sampling and filtration unit (104).

In an embodiment of the invention; the urea sensing unit (100), phosphate sensing unit (101), pH sensing unit (102), reference unit (103) and sampling and filtration unit (104) are positioned between two plastic layers (105).

In an embodiment of the invention; the urea sensing unit (100), phosphate sensing unit (101), pH sensing unit (102) and reference unit (103) are positioned at the lower side of the sampling and filtration unit (104).

In an embodiment of the invention; one removable plastic cover (106), urea sensing unit (100), phosphate sensing unit (101), pH sensing unit (102) and sampling and filtration unit (104) are positioned between two plastic layers (105).

Two plastic layers (105) provide insulation. However, the part at the side of sampling and filtration unit (104) is left open.

The removable plastic cover (106) is designed as it acts like a cap on this part whose top is open. Before the measurement process with the blood sample is started, it is removed and thrown away.

The sensing units (100, 101 and 102) perform reading from a little amount of blood sample which is taken from finger and dropped onto the sampling and filtration unit (104).

The sensing units (100, 101 and 102) which are to perform said measurements comprise polymer, ionophore, plasticizer, modified graphite, modified graphene, ionic resistance reducer and polymeric membrane which has selectivity increasing materials and composite membrane structures.

The disclosed urea, phosphate and pH measuring device (1) of the invention comprises a control module (20) in which the data received in consequence of the measurement from the blood sample is processed.

The control module (20) comprises a control unit (200) which evaluates the data that it receives from the urea sensing unit (100) determining the urea value in blood, from the phosphate sensing unit (101) determining the phosphate value and from the pH sensing unit (102) determining pH value; a display (201) which shows the values to the patient or user and a power unit (202). The power unit (202) may also be added from outside according to the embodiment of the invention.

The control module (20) comprises a control unit (200) which provides that emergency information is given to the user if the values measured are lower or higher in a predetermined amount than the value interval that healthy humans need to have.

The control unit (200) compares the data that it receives from the urea sensing unit (100), phosphate sensing unit (101) and pH sensing unit (102) with the reference values and transmits to the user if the urea, phosphate and pH values in his blood is in a normal value interval.

The normal values mean the urea, phosphate amount that healthy humans need to have in their blood and the pH of the blood of healthy humans.

For example; the phosphate level that needs to be in normal human blood is between 2,5-4,8 mg/dl. If the phosphate value is calculated by the control unit (200) at said interval in consequence of the measurement, the disclosed device (1) of the invention will transmit that the phosphate value is normal to the patient via the display (201).

In an embodiment of the invention; the control unit (200) comprises a micro processor or micro controller unit, an analog to digital converter (ADC), a channel multiplexor (MUX), an operational amplifier (Op-Amp).

Connecting the sensing units (100, 101 and 102) which have very high impedance to the ADC integrated circuits which have a low input impedance is provided via the control unit (200).

Output signals of the sensing units (100, 101 and 102) which are converted to a digital magnitude are sent to the control module (20) via a transmitter module.

In an embodiment of the invention, the control module (20) can connect to a smart device via bluetooth wirelessly.

The display (201) is a screen that provides transmission of the data to the user. The user can make all necessary adjustments by using this screen and monitor the data via menus on the screen.

The power unit (202) is a unit which provides the necessary energy for working of the urea, phosphate and pH measuring device (1).

The disclosed urea, phosphate and pH measuring device (1) of the invention comprises a communication module (30) which the data received from blood sample in consequence of the measurement is processed into.

The communication module (30) provides transmission of the data obtained in consequence of the measurement to the patient and/or related doctor.

In an embodiment of the invention, the communication unit (203) comprises a sim card which provides the transmission of the results to the patient and/or doctor.

The disclosed urea, phosphate and pH measuring device (1) of the invention has a size such that it can be carried easily.

The measurement device (1) monitors also the pH value together with the phosphate and urea amount. In an embodiment of the invention; urea, phosphate and pH measurement in 5-10 microliters of a blood sample is performed via the urea sensing unit (100), phosphate sensing unit (101) and pH sensing unit (102) in the strip type measurement module (10).

Therefore; a safe and effective monitoring which can be used by the patient at home easily and has high originality, sensitivity and accuracy percentage is expected in early detection of the uremic syndrome and hyperphosphatemia in renal failure patients.

The invention is not limited with the disclosed embodiments above, a skilled person in the art can produce different embodiments of the invention easily. They should be evaluated within the scope of invention protection demanded with claims.

## Claims

1. A potentiometric measurement device (1) **comprising** a measurement module (10) comprising only one reference unit (103), at least one urea sensing unit (100) for determining the urea amount in blood correctly, at least one phosphate sensing unit (101) for determining the phosphate amount in blood, at least one pH sensing unit (102) for determining the hydronium ion level in blood, and a sampling and filtration unit (104) which the blood sample whose urea, phosphate and pH values are to be measured is dropped into, wherein the urea sensing unit (100), phosphate sensing unit (101), pH sensing unit (102) and reference unit (103) are positioned at the lower side of the sampling and filtration unit (104), wherein the sampling and filtration unit (104) comprises a calibration solution and wherein reference unit (103) comprises only one reference electrode which is connected in series with the urea sensing unit (100), phosphate sensing unit (101), pH sensing unit (102) and showing constant potential in the sample solution for determination of the urea, phosphate concentration in the blood of patient and the pH of the blood, the sampling and filtration unit (104) is only one common sampling and filtration unit (104) for the urea sensing unit (100), the phosphate sensing unit (101), the pH sensing unit (102) and the reference unit (103).

2. A measurement device (1) according to claim 1; **wherein it comprises** a control module (20) which determines the urea, phosphate and pH value in blood dropped into a sampling and filtration unit (104), via the data measured by the urea sensing unit (100), phosphate sensing unit (101), pH sensing unit (102) and a reference unit (103) comprising only one reference electrode showing constant potential in the sample solution for determination of the urea, phosphate concentration in the blood of patient and the pH of the blood, and provides comparing said data with the reference values of healthy humans and presentation to the user.

3. A measurement device (1) according to claim 1 **wherein it comprises** a measurement module (10) which can measure the urea, phosphate and pH value from 5-10 microliters of blood such that it can be taken from a finger tip.

4. A measurement device (1) according to claim 1 or 2 **wherein it comprises** a control module (20) which determines the urea, phosphate and pH value in blood at the same time and provides comparing said data with reference values and presentation to the user at the same time.

5. A measurement device (1) according to claim 4 **wherein it comprises** the control module (20) which has a control unit (200) that presents to the user if the measurement values which it takes from the urea sensing unit (100), phosphate sensing unit (101) and pH sensing unit (102) are in the interval that healthy humans need to have by comparing them with the reference values and how much they are lower or higher if they are not in the value interval that healthy humans need to have by controlling them.

6. A measurement device (1) according to claim 4 **wherein it comprises** the control module (20) having the control unit (200) which provides that emergency information is given to the user if the values measured are lower or higher in a predetermined amount than the value interval of healthy humans.

7. **A** measurement device **(1)** according to claim **6 wherein it comprises** a measurement module (10) which comprises at least one urea sensing unit (100), at least one phosphate sensing unit **(101)**, at least one pH sensing unit **(102)** and at least one reference unit (103).

8. **A** measurement device (1) according to claim 7 **wherein it comprises** the measurement module (10) which comprises a strip type sampling and filtration unit (104) which a calibration solution is provided to be absorbed by and in which the blood sample is to be positioned.

9. **A** measurement device (1) according to claim 6 or 7 **wherein it comprises** at least one urea sensing unit (100) which provides determination of the urea concentration in blood by sensing the changes of the ammonium ion that is formed as a result of the urease catalyzed hydrolysis reaction.

10. **A** measurement device (1) according to claim 9 **wherein it comprises** the urea sensing unit (100) which comprises at least one urea biosensor having the urease enzyme for determination of the urea amount in blood.

11. **A** measurement device (1) according to claim 7 **wherein it comprises** the phosphate sensing unit (101) which is sensitive to HPO₄²⁻ (hydrogen phosphate) ion for determination of the phosphate amount in blood.

12. **A** measurement device (1) according to claim 11 **wherein it comprises** the phosphate sensing unit (101) which has at least one composite structured sensor comprising ionophore that is sensitive to HPO₄²⁻ (hydrogen phosphate), epoxy, hardener and modified graphite or graphene for determination of the phosphate amount in blood.

13. **A** measurement device (1) according to claim 7 **wherein it comprises** at least one pH sensing unit (102) which can measure the pH value for determination of acidity or alkalinity of the patient's blood, is necessary for correction of the urea and phosphate amounts at the same time.

14. **A** measurement device **(1)** according to claim **1 wherein it comprises** a communication unit (203) which provides transmission of the data obtained in consequence of the measurements to the patient and/or doctor wirelessly by providing remote access via a smart device after the data received from the measurement module (10) are evaluated and calculated by the control module (20).

15. **A** measurement device **(1)** according to claim **1** or **14 wherein it comprises** a display (201) which shows the interpreted values obtained in consequence of the measurement to the patient and/or doctor.

## Patentansprüche

1. Potentiometrische Messvorrichtung (1) mit einem Messmodul (10) mit nur einer Referenzeinheit (103), wenigstens einer Harnstoff-Messeinheit (100) zur korrekten Bestimmung des Harnstoffgehalts von Blut, wenigstens einer Phosphat-Messeinheit (101) zur Bestimmung des Phosphatgehalts im Blut, wenigstens einer pH-Messeinheit (102) zur Bestimmung der Konzentration von Wasserstoffionen im Blut und einer Probennahme- und Filtrations-Einheit (104), in die die Blutprobe, deren Harnstoff-, Phosphat- und pH-Werte zu messen ist, getropft wird, wobei die Harnstoff-Messeinheit (100), die Phosphat-Messeinheit (101), die pH-Messeinheit (102) und die Referenzeinheit (103) an der unteren Seite der Probennahme- und Filtrations-Einheit (104) positioniert sind, wobei die Probennahme- und Filtrations-Einheit (104) eine Kalibrationslösung enthält und wobei die Referenzeinheit (103) nur eine Referenzelektrode aufweist, die in Reihe mit der Harnstoff-Messeinheit (100), der Phosphat-Messeinheit (101), der pH-Messeinheit (102) verbunden ist und die konstantes Potential in der Probenlösung zur Bestimmung von Harnstoff-, Phosphat-Konzentration in dem Blut des Patienten und des pH-Wertes des Bluts zeigt, wobei die Probennahme- und Filtrations-Einheit (104) nur eine gemeinsame Probennahme- und Filtrations-Einheit (104) für die Harnstoff-Messeinheit (100), die Phosphat-Messeinheit (101), die pH-Messeinheit (102) und die Referenzeiheit (103) ist.

2. Messvorrichtung (1) nach Anspruch 1, wobei diese ein Steuermodul (20) aufweist, das den Harnstoff-, Phosphat- und pH-Wert von in eine Probennahme- und Filtrations-Einheit (104) getropftem Blut über die Daten bestimmt, die von der Harnstoff-Messeinheit (100), der Phosphat-Messeinheit (101), der pH-Messeinheit (102) und einer Referenzeinheit (103) mit nur einer Referenzelektrode gemessen worden sind, die konstantes Potential in der Probenlösung zur Bestimmung von Harnstoff-, Phosphatkonzentration in dem Blut des Patienten und des pH-Wertes des Blutes zeigt, und die für einen Vergleich der Daten mit Referenzwerten von gesunden Menschen und für die Präsentation an den Benutzer sorgt.

3. Messvorrichtung (1) nach Anspruch 1, wobei diese ein Messmodul (10) aufweist, das die Harnstoff-, Phosphat- und pH-Werte von 5-10 Mikrolitern Blut messen kann, so dass dieses von einer Fingerspitze abgenommen werden kann.

4. Messvorrichtung (1) nach Anspruch 1 oder 2, wobei diese ein Steuermodul (20) aufweist, das die Harnstoff-, Phosphat- und pH-Werte im Blut gleichzeitig bestimmt und gleichzeitig für den Vergleich der Daten mit Bezugswerten und die Präsentation für den Benutzer sorgt.

5. Messvorrichtung (1) nach Anspruch 4, wobei diese das Steuermodul (20) umfasst, das eine Steuereinheit (200) hat, die den Benutzer informiert, wenn die von der Harnstoff-Messeinheit (100), der Phosphat-Messeinheit (101) und der pH-Messeinheit (102) aufgenommenen Daten in dem Intervall liegen, das gesunde Menschen haben sollten, indem diese mit Referenzwerten verglichen werden, und darüber informiert, um wie viel sie niedriger oder höher sind, wenn sie nicht in dem Werteintervall liegen, das gesunde Menschen haben sollten, indem sie kontrolliert werden.

6. Messvorrichtung (1) nach Anspruch 4, wobei diese das Steuermodul (20) mit der Steuereinheit (200) umfasst, die dafür sorgt, dass dem Benutzer Notfallinformationen gegeben werden, wenn die gemessenen Werte um einen vorgegebenen Betrag niedriger oder höher als das Werteintervall für gesunde Menschen sind.

7. Messvorrichtung (1) nach Anspruch 6, wobei diese ein Messmodul (10) aufweist, das wenigstens eine Harnstoff-Messeinheit (100), wenigstens eine Phosphat-Messeinheit (101), wenigstens eine pH-Messeinheit (102) und wenigstens eine Referenzeinheit (103) aufweist.

8. Messvorrichtung (1) nach Anspruch 7, wobei diese das Messmodul (110) mit einer Probennahme- und Filtrations-Einheit (104) vom Streifentyp aufweist, die dazu vorbereitet ist, eine Kalibrationslösung zu absorbieren und in die die Blutprobe zu füllen ist.

9. Messvorrichtung (1) nach Anspruch 6 oder 7, wobei diese wenigstens eine Harnstoff-Messeinheit (100) aufweist, die die Bestimmung der Harnstoffkonzentration im Blut durchführt, indem Veränderungen der Ammoniumionen erfasst werden, die als Resultat der Urease-katalysierten Hydrolysereaktion gebildet werden.

10. Messvorrichtung (1) nach Anspruch 9, wobei diese die Harnstoff-Messeinheit (100) umfasst, die wenigstens einen Harnstoff-Biosensor mit einem Urease-Enzym zur Bestimmung des Harnstoffgehalts im Blut hat.

11. Messvorrichtung (1) nach Anspruch 7, wobei diese die Phosphat-Messeinheit (101) umfasst, die sensitiv ist auf das HPO₄²⁻-Ion (Hydrogenphosphat) zur Bestimmung des Phosphatgehalts im Blut.

12. Messvorrichtung (1) nach Anspruch 11, wobei diese die Phosphat-Messeinheit (101) aufweist, die wenigstens einen Sensor mit Kompositstruktur hat, die ein lonophor, das sensitiv auf HPO₄²⁻ (Hydrogenphosphat) ist, Epoxid, Härtungsmittel und modifiziertes Graphit oder Graphen zur Bestimmung des Phosphatgehalts im Blut aufweist.

13. Messvorrichtung (1) nach Anspruch 7, wobei diese wenigstens eine pH-Messeinheit (102) aufweist, die den pH-Wert zur Bestimmung des Säuregrades oder der Alkalität des Blutes des Patienten messen kann, die zur Korrektur von gleichzeitig Harnstoffgehalt und Phosphatgehalt notwendig sind.

14. Messvorrichtung (1) nach Anspruch 1, wobei diese eine Kommunikationseinheit (203) aufweist, die für die drahtlose Übertragung der infolge der Messungen erhaltenen Daten zu dem Patienten und/oder Arzt sorgt, indem Fernzugriff durch ein intelligentes Gerät ermöglicht wird, nachdem die von dem Messmodul (10) empfangenen Daten durch das Steuermodul (20) bewertet und berechnet sind.

15. Messvorrichtung (1) nach Anspruch 1 oder 14, die eine Anzeige (201) aufweist, die infolge der Messung erhaltene, interpretierte Werte dem Patienten und/oder dem Arzt anzeigt.

## Revendications

1. Dispositif de mesure potentiométrique (1) comprenant un module de mesure (10) comprenant seulement une unité de référence (103), au moins une unité de détection d'urée (100) pour déterminer la quantité d'urée dans le sang correctement, au moins une unité de détection de phosphate (101) pour déterminer la quantité de phosphate dans le sang, au moins une unité de détection de pH (102) pour déterminer le niveau d'ions hydronium dans le sang et une unité d'échantillonnage et de filtration (104) dans laquelle tombe goutte à goutte l'échantillon de sang dont les valeurs d'urée, de phosphate et de pH doivent être mesurées, dans lequel l'unité de détection d'urée (100), l'unité de détection de phosphate (101), l'unité de détection de pH (102) et l'unité de référence (103) sont positionnées au niveau du côté inférieur de l'unité d'échantillonnage et de filtration (104), dans lequel l'unité d'échantillonnage et de filtration (104) comprend une solution d'étalonnage et dans lequel l'unité de référence (103) comprend seulement une électrode de référence qui est connectée en série à l'unité de détection d'urée (100), à l'unité de détection de phosphate (101), à l'unité de détection de pH (102) et présentant un potentiel constant dans la solution d'échantillon pour la détermination de la concentration d'urée et de phosphate dans le sang du patient et du pH du sang, l'unité d'échantillonnage et de filtration (104) est seulement une unité d'échantillonnage et de filtration commune (104) pour l'unité de détection d'urée (100), l'unité de détection de phosphate (101), l'unité de détection de pH (102) et l'unité de référence (103).

2. Dispositif de mesure (1) selon la revendication 1 ; dans lequel il comprend un module de commande (20) qui détermine les valeurs d'urée, de phosphate et de pH dans le sang qui est tombé goutte à goutte dans une unité d'échantillonnage et de filtration (104) via les données mesurées par l'unité de détection d'urée (100), l'unité de détection de phosphate (101), l'unité de détection de pH (102) et une unité de référence (103) comprenant seulement une électrode de référence montrant un potentiel constante dans la solution d'échantillon pour la détermination de la concentration d'urée et de phosphate dans le sang du patient et du pH du sang, et fournit une comparaison desdites données avec les valeurs de référence d'humains en bonne santé et une présentation à l'utilisateur.

3. Dispositif de mesure (1) selon la revendication 1, dans lequel il comprend un module de mesure (10) qui peut mesurer les valeurs d'urée, de phosphate et de pH à partir de 5-10 microlitres de sang de sorte qu'il puisse être prélevé d'un bout de doigt.

4. Dispositif de mesure (1) selon la revendication 1 ou 2, dans lequel il comprend un module de commande (20) qui détermine les valeurs d'urée, de phosphate et de pH dans le sang en même temps et fournit une comparaison desdites données à des valeurs de référence et une présentation à l'utilisateur en même temps.

5. Dispositif de mesure (1) selon la revendication 4, dans lequel il comprend le module de commande (20) qui présente une unité de commande (200) qui montre à l'utilisateur si les valeurs de mesure qu'il prélève de l'unité de détection d'urée (100), de l'unité de détection de phosphate (101) et de l'unité de détection de pH (102) se situent dans l'intervalle dont les humains en bonne santé ont besoin en les comparant aux valeurs de référence et de combien elles sont inférieures ou supérieures si elles ne se trouvent pas dans l'intervalle de valeurs que les humains en bonne santé doivent présenter en les contrôlant.

6. Dispositif de mesure (1) selon la revendication 4 dans lequel il comprend le module de commande (20) présentant l'unité de commande (200) qui fournit des informations d'urgence qui sont présentées à l'utilisateur si les valeurs mesurées sont inférieures ou supérieures dans une quantité prédéterminée à l'intervalle de valeurs d'humains en bonne santé.

7. Dispositif de mesure (1) selon la revendication 6, dans lequel il comprend un module de mesure (10) qui comprend au moins une unité de détection d'urée (100), au moins une unité de détection de phosphate (101), au moins une unité de détection de pH (102) et au moins une unité de référence (103).

8. Dispositif de mesure (1) selon la revendication 7, dans lequel il comprend le module de mesure (10) qui comprend une unité d'échantillonnage et de filtration du type à bande (104) à laquelle une solution d'étalonnage est fournie pour y être absorbée et dans lequel l'échantillon de sang doit être positionné.

9. Dispositif de mesure (1) selon la revendication 6 ou 7, dans lequel il comprend au moins une unité de détection d'urée (100) qui fournit une détermination de la concentration d'urée dans le sang en détectant les changements de l'ion ammonium qui est formé à la suite de la réaction d'hydrolyse catalysée par l'uréase.

10. Dispositif de mesure (1) selon la revendication 9, dans lequel il comprend l'unité de détection d'urée (100) qui comprend au moins un biocapteur d'urée présentant l'enzyme uréase pour la détermination de la quantité d'urée dans le sang.

11. Dispositif de mesure (1) selon la revendication 7, dans lequel il comprend l'unité de détection de phosphate (101) qui est sensible à l'ion HPO₄²⁻ (phosphate d'hydrogène) pour la détermination de la quantité de phosphate dans le sang.

12. Dispositif de mesure (1) selon la revendication 11, dans lequel il comprend l'unité de détection de phosphate (101) qui présente au moins un capteur de structure composition comprenant un ionophore qui est sensible au HPO₄²⁻ (phosphate d'hydrogène), une résine époxyde, un durcisseur et du graphite ou du graphène modifié pour la détermination de la quantité de phosphate dans le sang.

13. Dispositif de mesure (1) selon la revendication 7, dans lequel il comprend au moins une unité de détection de pH (102) qui peut mesurer la valeur du pH pour déterminer l'acidité ou l'alcalinité du sang du patient et qui est nécessaire pour la correction des quantités d'urée et de phosphate en même temps.

14. Dispositif de mesure (1) selon la revendication 1, dans lequel il comprend une unité de communication (203) qui fournit une transmission des données obtenues à la suite des mesures au patient et/ou au médecin sans câble en fournissant un accès à distance via un dispositif intelligent une fois que les données reçues du module de mesure (10) ont été évaluées et calculées par le module de commande (20).

15. Dispositif de mesure (1) selon la revendication 1 ou 14, dans lequel il comprend un appareil d'affichage (201) qui montre les valeurs interprétées obtenues à la suite de la mesure au patient et/ou au médecin.
